# EUROPEAN PATENT SPECIFICATION

(11) **EP 4 406 496 B1**
(45) Date of publication and mention of the grant of the patent: **03.12.2025**
(21) Application number: 22886030.0
(22) Date of filing: 26.10.2022
(51) Int. Cl.: A61B 17/22, A61M 25/10

(54) **ELECTRODE BALLOON CATHETER**
ELEKTRODENBALLONKATHETER
CATHÉTER À BALLONNET À ÉLECTRODE

(30) Priority: 27.10.2021 CN 202111257836
(43) Date of publication of application: 31.07.2024
(73) Proprietor: Shanghai Microport Rotapace Medtech Co., Ltd., Shanghai 201203 (CN)
(72) Inventor: JI, Xiaofei, Shanghai 201203 (CN); CHANG, Zhaohua, Shanghai 201203 (CN); YUE, Bin, Shanghai 201203 (CN); YAO, Yingzhong, Shanghai 201203 (CN)
(74) Representative: Marks & Clerk LLP
(86) International application number: PCT/CN2022/127730
(87) International publication number: WO 2023/072154

(56) References cited:
- CN-A- 104 736 073
- CN-A- 112 220 526
- CN-A- 112 869 826
- CN-A- 113 842 190
- CN-B- 112 220 526
- CN-U- 214 907 695
- CN-U- 216 167 694
- US-A1- 2014 163 592
- US-A1- 2020 397 453
- US-A1- 2022 218 402

## Description

### TECHNICAL FIELD

The present invention relates to the field of medical devices and, in particular, to an electrode balloon catheter.

### BACKGROUND

With the continuous development of percutaneous coronary intervention (PCI), this therapy is indicated for an increasing number of more and more complex lesions. Calcified coronary lesions, in particular those with a high degree of calcification, or complex ones with tortuosity, calcified nodules and infiltration, have been challenging and risky for interventional therapy. Correct identification and assessment of a calcified lesion and choice of a suitable interventional treatment technique are critical to more likely success, reduced complications and improved short- and long-term patient prognosis.

Conventionally, a calcified lesion is often treated by dilation with a balloon. This, however, tends to cause balloon-induced barotrauma, which may lead to intimal disruption, thrombosis, vascular restenosis and other issues. Shockwave balloon catheters based on the electrohydraulic effect can crush fibrotic and calcified plaques in blood vessels, effecting rapid disruption and dilatation of calcified lesions. A shockwave balloon catheter works by generating high-voltage pulsed electric fields each lasting for a short period of time (<10 ms) within a blood vessel, creating high-pressure sound waves. When these high-energy sound waves propagate to a calcified plaque on the wall of the blood vessel, stress resulting from impingement of the shock waves will induce cracks in the plaque, just like how they break apart anything fragile. Under the action of repeated impingement of such shock waves, the existing cracks will be enlarged, until the calcified plaque is broken up. This technique allows low-pressure dilatation of a calcified lesion, avoiding the problem of possible damage to the wall of the blood vessel caused by sudden excessive dilatation that may arise from the use of a high-pressure balloon in conventional angioplasty.

In most existing shockwave balloon catheters, electrodes are arranged on the catheter body and therefore far away from the exterior of the balloon body. However, the energy density of shock waves diminishes exponentially as a function of the distance they propagate. Therefore, the large spacing would greatly reduce the effectiveness of shock waves for diseased tissue, requiring extending the duration of the surgical procedure and increasing cycles of release from the electrodes. Consequently, ischemic complications tend to occur due to dilatation of the blood vessel and occlusion of blood flow for a prolonged period of time. Moreover, the increased number of shockwave release cycles imposes more stringent requirements on the service life of the electrodes, eventually affecting the material and size of the electrodes and the crossability of the catheter, and increasing the cost of fabrication. Additionally, as it is challenging for existing shockwave balloon catheters to deal with severely eccentric lesions, they are not suited to use in targeted treatment of asymmetrically calcified lesions. Thus, despite demonstrated excellent therapeutic outcomes, shockwave balloon catheters based on the electrohydraulic effect have been found with deficiencies, in particular a large distance from the shockwave source to the target lesion and poor performance in targeted treatment of asymmetrically calcified lesions. US2014163592A1 discloses a catheter for use in valvuloplasty including an inflatable balloon carried by an elongated body. A shock wave source is provided within the inflatable balloon. A follower arrangement is provided that maintains the shock wave source a substantially fixed distance from the inner surface of the balloon. CN112220526B discloses a pulse balloon comprising a guidewire cavity in a catheter body, a sealing tip at one end of the catheter body, a first balloon arranged around the catheter body at the sealing tip, and the first balloon surrounding the catheter body to form an annular channel. US2020397453A1 discloses a photoacoustic catheter with an elongate shaft, a balloon and a photoacoustic transducer.

### SUMMARY OF THE INVENTION

The invention is defined by independent claim 1, with further embodiments defined by the dependent claims.

In order to solve the problems with the prior art, the present invention provides an electrode balloon catheter that allows for the adjustment of the position of the electrodes so as to adjust a propagation distance for shock waves. This allows more efficient lesion treatment, reduced surgical duration and an extended service life of the electrodes. Moreover, it has the advantages of a simple structure and ease of surgical operation.

To this end, the electrode balloon catheter comprises:
a catheter body;
an outer balloon, wherein the outer balloon is disposed at an end of the catheter body and is configured to store a conductive medium;
an inner balloon, wherein the inner balloon is disposed at the end of the catheter body and located inside the outer balloon; and
at least one electrode pair configured to receive high-voltage pulses and thereby generate shock waves, wherein at least one electrode pair is disposed on a surface of the inner balloon, wherein the electrode pair comprises a positive electrode and a negative electrode, wherein the positive electrode and the negative electrode are insulated from each other, and wherein an insulating distance between the positive electrode and the negative electrode is fixed or variable, and wherein the insulating distance between the positive and negative electrodes is configured within a predetermined range,
wherein the outer balloon, when inflated, is configured to fit onto a target object, wherein the inner balloon is configured to bring the electrode pair closer to or farther away from the target object through an inflation of the inner balloon, and wherein the electrode pairs can be adjusted in position with respect to the target lesion by modifying a degree of inflation, and hence a diameter of the inner balloon.

Optionally, at least one electrode pair may be disposed on an outer surface of the inner balloon.

Optionally, the predetermined range may be from 0.01 mm to 10 mm.

Optionally, the positive electrode and the negative electrode are rigidly connected, flexibly connected or not connected.

Optionally, the electrode pair may comprise two electrodes with opposite polarities, wherein each electrode is formed on the surface of the inner balloon by electroplating or is provided in the form of a flexible circuit.

Optionally, the electrode balloon catheter may comprise a plurality of electrode pairs, wherein the plurality of electrode pairs are arranged axially along and circumferentially around the inflated inner balloon.

Optionally, the positive electrode and the negative electrode of each electrode pair are arranged axially along the inflated inner balloon, wherein the positive and negative electrodes in adjacent electrode pairs are connected by electrical leads.

Optionally, the inner balloon may be made of a compliant material or a non-compliant material.

Optionally, the catheter body may comprise an inner tube and an outer tube, the inner tube received in the outer tube and protruding out of a distal end of the outer tube, wherein: each of a proximal end and a distal end of the inner balloon is fixedly attached to the inner tube; a proximal end of the outer balloon is fixedly attached to the outer tube; a distal end of the outer balloon is fixedly attached to the inner tube; an outer-balloon fluid supply lumen in communication with the outer balloon is formed between the inner and outer tubes; and an inner-balloon fluid supply lumen in communication with the inner balloon is provided within the inner tube.

Optionally, the catheter body may further comprise a handle that is located at a proximal end, wherein the proximal end of the inner and the proximal end of outer tube are connected to the handle, the handle provided with an outer-balloon fluid inlet and an inner-balloon fluid inlet, the outer-balloon fluid inlet connected to the outer-balloon fluid supply lumen, the inner-balloon fluid inlet connected to the inner-balloon fluid supply lumen.

Optionally, an inflated outer balloon may have a diameter of 0.75 mm to 30.0 mm and an axial length of 3 mm to 300 mm.

Optionally, an inflated inner balloon may have a diameter of 0.5 mm to 29.0 mm and an axial length of 3 mm to 300 mm.

In the electrode balloon catheter as defined above, through positioning the electrode pairs on the inner balloon, their positions relative to a target lesion can be adjusted by inflating or deflating the inner balloon, thereby changing a propagation distance for shock waves and their impingement energy on the target lesion. This can enhance lesion treatment efficiency, reduce the surgical duration and extend the service life of the electrodes. Moreover, in the electrode balloon catheter, adjustability in position of the electrode pairs through manipulating the balloon can be achieved by a simple structure and can provide ease of surgical operation.

In the electrode balloon catheter as defined above, if the electrode pairs are provided on the outer surface of the inner balloon, the electrodes can come into direct contact with the conductive medium, imparting greater directionality to the propagation of shock waves. As a result, shock waves can more effectively radiate outwardly towards the outer surface of the outer balloon, resulting in an increase in lesion treatment efficiency. Otherwise, if the electrodes are disposed on the inner surface of the inner balloon, they can be protected by both balloons and is therefore safer.

In the electrode balloon catheter as defined above, the positive and negative electrodes in each electrode pair may be insulated from each other at a fixed or variable insulating distance, adding more flexibility to lesion treatment. For example, when the inner balloon is made of a compliant material, the insulating distance will vary as a result of inflating the inner balloon. This allows the intensity of energy released from pulsed electric fields to be adjusted in a flexible manner and results in higher lesion treatment efficiency.

In the electrode balloon catheter as defined above, the electrode can be directly formed on the surface of the inner balloon by electroplating, enabling easier assembly of the electrodes. Moreover, the electrodes can have a reduced thickness, which allows the catheter to have a smaller outer diameter during crossing. Alternatively, the electrodes may be provided in the form of flexible circuits. In this case, the electrodes will be more flexible and easier to fold and also allow a smaller outer diameter during crossing.

In the electrode balloon catheter as defined above, a plurality of electrode pairs may be incorporated, which are arranged both axially along and circumferentially around the inflated inner balloon. In this case, in order to enable the balloon to have a smaller outer diameter during crossing, the electrode pairs are preferred to be connected in series by electrical leads. Specially, the positive and negative electrodes in each electrode pairs may be arranged axially along the inflated inner balloon, and positive and negative electrodes in adjacent electrode pairs may be connected by electrical leads.

### BRIEF DESCRIPTION OF THE DRAWINGS

Fig. 1 is a schematic diagram showing the structure of an electrode balloon catheter according to an embodiment of the present invention, in which an outer balloon has been inflated, while an inner balloon has not been inflated yet.
Fig. 2 is an enlarged view of a portion of the electrode balloon catheter of Fig. 1.
Fig. 3 is a schematic cross-sectional view of an electrode balloon catheter according to an embodiment of the present invention, in which an outer balloon has been inflated, while an inner balloon has not been inflated yet.
Fig. 4 is a schematic diagram showing the structure of an electrode balloon catheter according to an embodiment of the present invention, in which both an outer balloon and an inner balloon have been inflated.
Fig. 5 is an enlarged view of a portion of the electrode balloon catheter of Fig. 4.
Fig. 6 is a schematic cross-sectional view of an electrode balloon catheter according to an embodiment of the present invention, in which both an outer balloon and an inner balloon have been inflated.
Fig. 7 is an unrolled view of an inflated inner balloon according to a preferred embodiment of the present invention.

In these figures,
1, a catheter body; 11, an inner tube; 111, an outer-balloon fluid supply lumen; 112, an inner-balloon fluid supply lumen; 12, an outer tube; 2, an outer balloon; 22, a tapered section; 21, a straight section; 3, an inner balloon; 4, an electrode pair; 5, an electrical lead; 6, a handle; 61, an outer-balloon fluid inlet; 62, an inner-balloon fluid inlet; and 63, an energy interface.

### DETAILED DESCRIPTION

The present disclosure will be apparent and readily understood from the following detailed description of specific embodiments of the invention taken in conjunction with the accompanying drawings. However, it will be understood that the present invention is not limited to the specific embodiments set forth below, and general alternatives known to those of skill in the art are intended to be also embraced in the scope thereof as long as they fall within the wording of the claims. Note that the figures are presented in a very simplified form not necessarily drawn to exact scale and for the only purpose of facilitating easy and clear description of the embodiments disclosed herein.

It would be appreciated that, in the following description, vertical spatial relationships of various elements may be described with reference to the orientation of the annexed figures. As used herein, the spatially relative terms "under", "below", "lower", "upper" and the like are used for ease of description to describe one element or feature's relationship to another element(s) or feature(s) as illustrated in the figures. The spatially relative terms are intended to encompass different orientations of the device in use or operation in addition to the orientation depicted in the figures. The device may be otherwise oriented, for example, rotated 90 degrees or at other orientations, and the spatially relative descriptors used herein interpreted accordingly. It would be further appreciated that, as used in the following description, the term "connection" and derivatives thereof may be used to refer to a direct connection between systems, components, or elements, or to a connection established by a medium intervening between systems, components, or elements, i.e., an indirect connection. As would be appreciated, as used in the following description, the use of the terms "first" and "second" herein is not meant to be limiting of the numerical number of the referenced item and is only intended to distinguish one component from another.

As used herein, the terms "proximal end" and "distal end" are employed to describe relative orientations, relative positions and directions between components of an electrode balloon catheter according to the present invention or actions thereof, as viewed by a surgeon using the device. "Proximal end" is usually used to describe an end farther away from a patient and closer to an operator, in contrast to "distal end" being usually used to describe an end closer to the patient and farther away from the operator, during normal operation of the electrode balloon catheter, although these terms are not intended to be limited to being used in such a way. The term "axial", "radial" and "circumferentially" may be used herein to describe directions parallel to, perpendicular to and about an axis, respectively. The term "plurality" is defined herein as two or more than two.

Electrode balloon catheters proposed in embodiments of the present invention will be described in detail below with reference to the accompanying drawings.

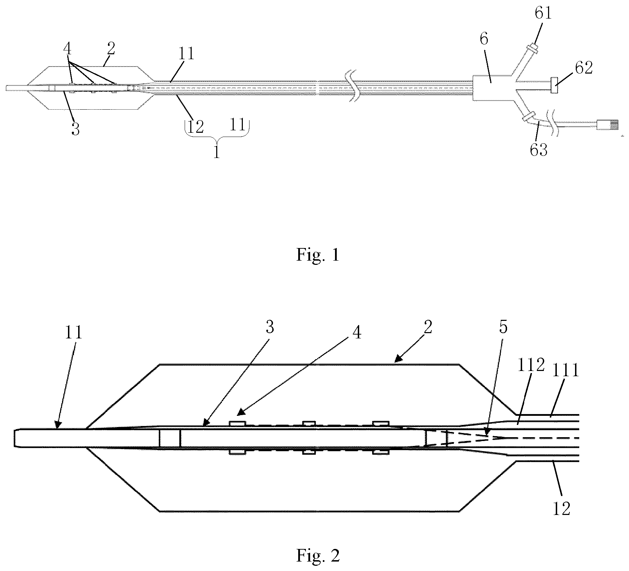

Referring to Figs. 1 to 2, the present invention provides an electrode balloon catheter including a catheter body 1, an outer balloon 2, an inner balloon 3 and electrode pairs 4. Both the outer balloon 2 and the inner balloon 3 are provided at an end of the catheter body 1. The inner balloon 3 is disposed inside the outer balloon 2 and configured to attach the electrode pairs 4 thereto. The interior of the outer balloon 2, which is defined as a space between the outer balloon 2 and the inner balloon 3, is configured to store a conductive medium for transmitting shock waves. At least one of the electrode pairs 4 is disposed on the inner balloon 3, e.g., on its inner or outer surface. When the electrode pairs 4 are disposed on the outer surface of the inner balloon 3, they can come into direct contact with the conductive medium, imparting greater directionality to the transmission of shock waves. As a result, shock waves can more effectively radiate outwardly towards an outer surface of the outer balloon 2. Otherwise, if the electrode pairs 4 are disposed on the inner surface of the inner balloon 3, they can be protected by both balloons and is therefore safer.

A plurality of electrode pairs 4 are provided, in which some may be provided on the inner surface of the inner balloon 3, and the remaining one(s) may be provided on outer surface of the inner balloon 3. Alternatively, all the electrode pairs 4 may be provided on the inner surface of the inner balloon 3, or on the outer surface thereof. As would be appreciated, the electrode pairs 4 are configured to accept high-voltage pulses for generating shock waves, and each electrode pair 4 includes one positive electrode and one negative electrode, which are insulated from each other at an insulating distance configured within a predetermined range. As would be appreciated, each electrode pair 4 is configured to generate pulsed electric fields between the positive and negative electrodes in the form of positive and negative electrode signals. If the insulating distance between the electrodes is not large enough, sparking may occur, or cold plasma may be generated. If the insulating distance is too large, the generated electric field intensity may be affected. For these reasons, the insulating distance between the two electrodes is designed within the predetermined range, which can ensure sufficient energy strength of the generated electric fields while not causing ionization. In this way, it can be ensured that desired energy can be delivered to a target lesion at a safe level. As also would be appreciated, the insulating distance refers to a distance between the positive and negative electrodes in a deflated or inflated configuration of the inner balloon 3, which varies in the course of deflation or inflation of the inner balloon 3 while always remaining within the predetermined range.

Use of the electrode balloon catheter of the present invention may involve inflating the outer balloon 2 so that it expands and fits onto a target calcified lesion in a blood vessel in a patient while surrounding the inner balloon 3 and the electrode pairs 4 to prevent the electrodes from coming into direct contact with tissue of the patient. Moreover, the electrode pairs 4 can be adjusted in position with respect to the target lesion by modifying a degree of inflation, and hence a diameter, of the inner balloon 3. In this way, a propagation distance for shock waves, and hence a level of energy of shock waves acting on the target lesion, may be modified, making the device suitable for use in more therapeutic treatment applications. In particular, when the inner balloon 3 is inflated, the electrode pairs 4 will be brought closer to the target lesion, reducing the propagation distance for shock waves. As a result, stronger shock waves with higher energy can be delivered to the target lesion to crush the calcified lesion more efficiently, thus reducing the required surgical duration and cycles of release of shock waves, and extending the service life of the electrodes. Further, when the target lesion is asymmetrically calcified, targeted treatment thereof can be achieved.

In Figs. 1 to 3, the outer balloon 2 is shown as being in an inflated configuration, and the inner balloon 3 in a non-inflated configuration. As shown in Figs. 1 to 3, the outer balloon 2, when fully inflated, can fit against and dilate the target lesion, and the electrode pairs 4 on the surface of the inner balloon 3 are distant from the outer balloon 2 in the collapsed configuration of the inner balloon 3. At this time, shock waves released from the electrode pairs 4 will propagate through the conductive medium in the outer balloon 2 to the outer balloon 2 and the wall of the blood vessel surrounding the outer balloon 2 and impinge on the calcified lesion. However, since the shock waves have to propagate a large distance, the energy density of them acting on the calcified lesion is relatively low.

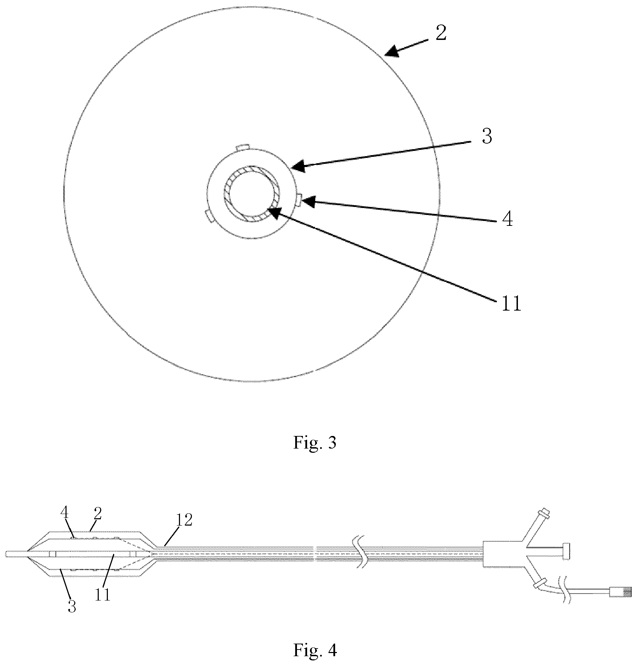

In Figs. 4 to 6, both the outer balloon 2 and the inner balloon 3 are shown as being in an inflated configuration. As shown in Figs. 4 to 6, when the inner balloon 3 is also inflated, the electrode pairs 4 on the surface of the inner balloon 3 will be brought closer to the surface of the outer balloon 2, as well as to the diseased tissue. At the same time, the distance from the electrode pairs 4 to the surface of the outer balloon 2 is reduced, i.e., the propagation distance for shock waves. As a result, energy loss arising from the propagation distance will be reduced, making it possible to more efficiently crush the lesion by stronger shock waves. As also would be appreciated, the inner balloon 3 may be inflated to a degree determined as required by the treatment of the lesion. The inner balloon 3 may be either fully inflated to a maximum diameter, or to a smaller diameter. In contrast, the outer balloon 2 is usually fully inflated so that it can fit against the blood vessel and dilate the vessel.

Therefore, through using the inflatable and collapsible inner balloon 3 in lieu of a conventional fixed electrode carrier, the electrodes in the electrode balloon catheter of the present invention is made freely movable within the outer balloon 2. This results in improved performance in crushing a calcified lesion, reduced surgical duration, a lower risk of causing ischemic complications, a reduced number of required cycles of shockwave release and an extended service life of the electrodes. In addition, in order to treat an asymmetrically calcified lesion, the electrode pairs 4 in the electrode balloon catheter of the present invention may be arranged on the surface of the inner balloon into alignment with the asymmetric lesion and then brought closer to the lesion as a result of inflating the inner balloon. In this way, targeted treatment of the asymmetrically calcified lesion can be achieved. Further, since fewer cycles of shockwave release are required, the requirements on material choice and dimensional design of the electrodes can be reduced, eventually allowing the electrode balloon catheter to have a smaller outer diameter during crossing, and the fabrication cost is reduced. Furthermore, the inner and outer balloons are typically folded during delivery. That is, both the inner balloon 3 and the outer balloon 2 are folded and crimped on the catheter body 1. In this configuration, the electrodes on the inner balloon 3 may be folded with the inner balloon 3. To this end, the electrodes may be provided, for example, in the form of very small patches or flexible circuits. The folded balloons allow a small outer diameter during crossing. The flexible circuits may be small, lightweight, thin, soft and flexible.

In practical therapeutic treatment applications, the number and arrangement of the electrode pairs 4 may be determined as required, as long as shock waves released from the electrodes can propagate to a target lesion in a blood vessel in a desired manner and achieve a desired therapeutic result. In this embodiment, a plurality of electrode pairs 4 are provided on the inner balloon 3, which are arranged axially along and/or circumferentially around the inner balloon 3. The number and positions of electrode pairs 4 that are circumferentially arranged determine a circumferential (or angular) impingement range of shock waves for a calcified lesion, and the number and positions of electrode pairs 4 that are axially arranged determine an axial (or lengthwise) impingement range of shock waves for a calcified lesion.

In Figs.1 to 6, 9 electrode pairs are shown, which are arranged both axially along and circumferentially around the inner balloon 3. It is a matter of course that the positive and negative electrodes in each electrode pair 4 are insulated from each other. The positive and negative electrodes in each electrode pair 4 may be insulated from each other at an insulating distance within a predetermined range, which is preferred to be 0.01 mm to 10 mm and more preferred to be 0.1 mm to 2.0 mm.

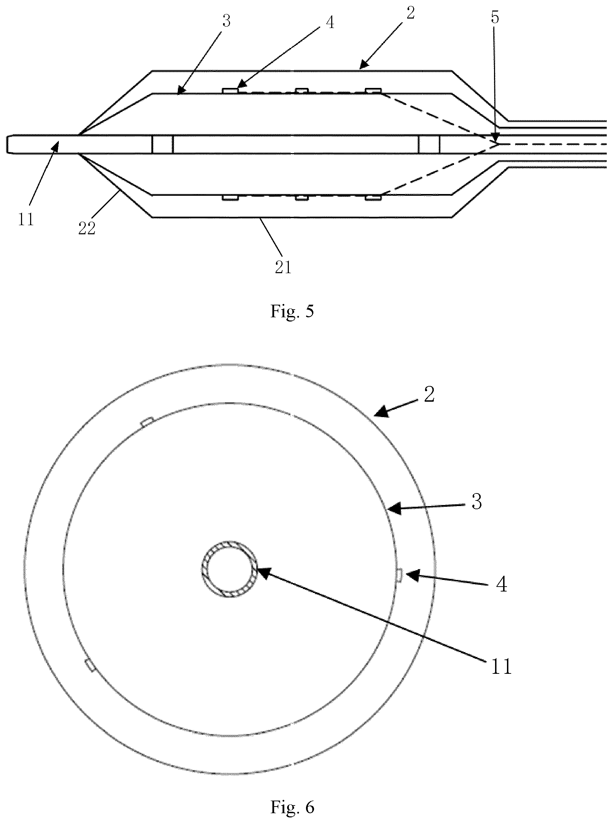

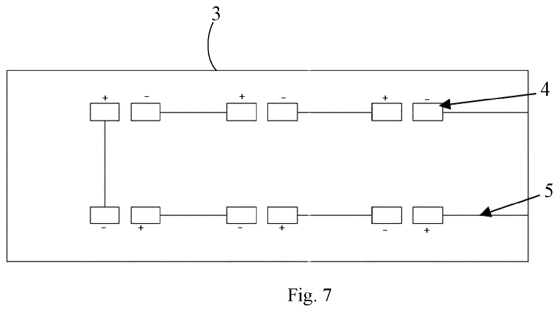

In Fig. 7, 6 electrode pairs 4 are shown, which are arranged both axially along and circumferentially around the inner balloon 3. Of course, the number of electrode pairs 4 is not limit to 6 or 9 as exemplified above. The plurality of electrode pairs 4 may be axially arranged either uniformly or not. Likewise, they may be circumferentially arranged either uniformly or not.

In some embodiments, the positive and negative electrodes in each electrode pair 4 are insulated from each other at a fixed insulating distance in order to ensure that consistent energy is released in each cycle. Accordingly, despite the variable propagation distance, shock waves with constant energy will be released from each electrode pair 4, and the insulating distance remains the same even when the inner balloon 3 vary in shape or size. In order to achieve such a fixed insulating distance, the positive and negative electrodes in each electrode pair 4 may be rigidly connected to each other and then fixed to the inner balloon 3 as a whole. Here, by "rigidly connected", it is intended to mean that when one of the positive and negative electrodes is displaced or stressed, the other electrode connected thereto will not displace or deform with respect to the first electrode.

In some embodiments, the positive and negative electrodes in each electrode pair 4 are insulated from each other at a variable insulating distance, which allows the intensity of energy delivered to be modified and adds flexibility to lesion treatment. Accordingly, as the propagation distance varies, the energy of shock waves released from each electrode pair 4 will change, e.g., increase or decrease, and the insulating distance will change in response to shape or size changes of the inner balloon 3. The degree of inflation, and hence the diameter, of the inner balloon 3 may be controlled to ensure that the variable insulating distance remains within the predetermined range. In order to achieve such a variable insulating distance, the positive and negative electrodes in each electrode pair 4 may be flexibly connected together and then fixed to the inner balloon 3 as a whole. Here, by "flexibly connected", it is intended to mean that when one of the positive and negative electrodes is displaced or stressed, the other electrode connected thereto may responsively displace or deform with respect to the first electrode. Alternatively, in order to achieve the variable insulating distance, the positive and negative electrodes in the electrode pairs 4 may be individually fixed to the inner balloon 3 without being connected together. As a result, the positive and negative electrodes can move freely and independently during inflation of the inner balloon 3.

This application is not limited to any particular method of fixing the electrode pairs 4 to the inner balloon 3. For example, the fixation may be accomplished by adhesive bonding, welding or electroplating. In order to achieve easier assembly, as well as reduced thickness and stiffness, of the electrodes, it is preferred that the electrodes are directly formed on the surface of the inner balloon 3 by electroplating, or made in the form of flexible circuits and attached to the inner balloon 3. In addition to easier assembly, fabricating the electrodes by electroplating allows the electrodes to have a smaller thickness and allows the electrode balloon catheter to have a reduced outer diameter during crossing. Providing the electrodes in the form of flexible circuits can facilitate their folding and also allows the electrode balloon catheter to have a reduced outer diameter during crossing.

As shown in Figs. 2 and 5, the electrode pairs 4 may be connected to electrical leads 5. Specifically, the positive electrodes in the electrode pairs 4 may be connected to a positive terminal of a power supply of a high-voltage pulse generator via electrical leads 5, and the negative electrodes in the electrode pairs 4 may be connected to a negative terminal of the power supply of the high-voltage pulse generator via electrical leads 5. The high-voltage pulse generator is configured to provide pulses required by the electrode pairs 4 for generating shock waves. In general, the high-voltage pulse generator may control the frequency and number of cycles of shockwave release from the electrodes by turning on and turning off a circuit.

This application is not limited to any particular type of electrodes. For example, patch or ring electrodes may be suitably used. A "ring electrode" may be shape like a ring that can be fitted over the inner balloon 3, and a "patch electrode" may be in the shape of a substantially flat or slightly curved patch that can be attached to the inner balloon 3. In this embodiment, the electrodes are patch electrodes designed based on the principles of point discharge, which can provide high energy density and enhance the electrohydraulic effect.

In addition, the application is not limited to any particular arrangement of the electrode pairs 4 on the inner balloon 3. They may be individually independent of one another, or connected in series or parallel.

In Fig. 7, six electrode pairs 4 are shown as being connected in series, as an illustrative example. The six electrode pairs 4 are arranged both axially along and circumferentially around the inflated inner balloon 3. For example, they may be arranged into two circumferential rows and three axial columns. The six electrode pairs 4 may be connected in series by electrical leads 5. More specifically, the negative electrode (labeled as "-" in Fig. 7) in each electrode pair 4 may be connected to the positive electrode (labeled as "+" in Fig. 7) in an adjacent electrode pair 4 via an electrical lead 5. As a result, a series circuit may be formed with a positive terminal connected to the positive terminal of the power supply of the high-voltage pulse generator and with a negative terminal connected to the negative terminal of the power supply of the high-voltage pulse generator. As would be appreciated, Fig. 7 shows the inner balloon 3 that has been inflated and then "unrolled". As can be seen in the unrolled plan view of the inflated inner balloon 3, the six electrode pairs 4 are connected in series by electrical leads 5, and the positive and negative terminals of the resulting series circuit are connected to the high-voltage pulse generator via electrical leads 5. This arrangement allows fewer electrical leads 5 to be used to connect the electrode pairs 4 and the high-voltage pulse generator. As a result, the catheter is allowed to have a reduced overall size and a smaller outer diameter during crossing. Of course, in other embodiments, the six electrode pairs 4 may be connected in parallel using electrical leads 5. Specifically, the positive electrode in each electrode pair 4 may be connected to the positive electrode in an adjacent electrode pair 4 via an electrical lead 5, and the negative electrode in each electrode pair 4 may be connected to the negative electrode in an adjacent electrode pair 4 via an electrical lead 5. As a result, a parallel circuit may be formed with a positive terminal connected to the positive terminal of the power supply of the high-voltage pulse generator and with a negative terminal connected to the negative terminal of the power supply of the high-voltage pulse generator. This arrangement also allows the use of fewer electrical leads. In further embodiments, the electrode pairs 4 may not be mutually connected by electrical leads 5. Instead, they may be independent of one another and separately connected to the high-voltage pulse generator. That is, the positive electrode in each electrode pair 4 may be connected to the positive terminal of the power supply of the high-voltage pulse generator via an electrical lead 5, and the negative electrode in each electrode pair 4 may be connected to the negative terminal of the power supply of the high-voltage pulse generator via an electrical lead 5.

This application is not limited to any particular material of the outer balloon 2. It may be made of a compliant or non-compliant material, preferably of a compliant material, because it can impart good crush resistance to the outer balloon 2 and enables it to better dilate a blood vessel. Moreover, it allows the balloon to be folded to an even smaller size, making it easier for the balloon to pass through, and hence dilate and treat, a narrow calcified lesion. A compliant material suitable for making the outer balloon 2 may be selected from polyurethane (PU), polyethylene (PE), silicone and other materials. A non-compliant material suitable for making the outer balloon 2 may be selected from polyethylene terephthalate (PET), nylon and other materials.

Figs. 2 and 5 show the outer balloon 2 in an inflated configuration. In this configuration, the outer balloon 2 may comprise a straight section 21 and tapered sections 22 joined to the straight section 21 at its opposite ends. In practical operation, the balloon dilates a blood vessel essentially by the straight section 21. Therefore, the straight section 21 can be considered as an effective section of the balloon. In practical applications, the outer balloon 2, when inflated, may have a diameter and axial length determined according to the size and extent of a target lesion to be treated. Optionally, the outer balloon 2 when inflated, may have a diameter in the range of 0.75 mm to 30.0 mm, preferably 2.0 mm to 20 mm, and an axial length in the range of 3 mm to 300 mm, preferably 4 mm to 250 mm.

Likewise, this application is not limited to any particular material of the inner balloon 3. It may be made of a compliant or non-compliant material. The inner balloon 3 may be made of the same compliant material as that of the outer balloon 2, which may be selected from, for example, PU, PE, silicone and other materials. Alternatively, the inner balloon 3 may be made of the same non-compliant material as that of the outer balloon 2, which may be selected from, for example, PET, nylon and other materials. When the material of the inner balloon 3 is selected as a non-compliant material, if the two electrodes in each electrode pair 4 are flexibly connected, then only minimal relative displacement may occur between them, which has a minor impact on their insulating distance and hence on the release of shock waves. Therefore, the insulating distance can be considered as constant. When the material of the inner balloon 3 is selected as a compliant material, if the two electrodes in each electrode pair 4 are flexibly connected, then relatively large displacement may occur between them, leading to a change in their insulating distance. Thus, it would be appreciated that when the material of the inner balloon 3 is non-compliant, the insulating distance between the two electrodes in each electrode pair 4 can be considered as constant, no matter whether they are rigidly connected, flexibly connected or not connected. In contrast, when the material of the inner balloon 3 is compliant, in order to achieve a constant insulating distance between the 2 electrodes in each electrode pair 4, these electrodes must be rigidly connected to each other. Likewise, in practical applications, the inner balloon 3, when inflated, may have a diameter and axial length determined according to the size and extent of a target lesion to be treated. Optionally, the inner balloon 3, when inflated, may have a diameter ranging from 0.5 mm to 29.0 mm, preferably from 0.7 mm to 28.0 mm, and an axial length ranging from 3 mm to 300 mm, preferably from 6 mm to 180 mm. Of course, the diameter of the inflated outer balloon 2 is greater than that of the inflated inner balloon 3. Moreover, the axial length of the inflated outer balloon 2 is greater than that of the inflated inner balloon 3.

In one exemplary embodiment, the insulating distance between the positive and negative electrodes in each electrode pair 4 is 0.7 mm. Moreover, the inner balloon 3 is allowed to be inflated to a maximum diameter of 2.5 mm, and the outer balloon 2 is allowed to be inflated to a maximum diameter of 3.0 mm.

Additionally, the catheter body 1 may comprise an outer-balloon fluid supply lumen 111 and an inner-balloon fluid supply lumen 112, as shown in Fig. 2. The outer-balloon fluid supply lumen 111 may communicate with the outer balloon 2 to allow a fluid (e.g., the conductive medium) to be supplied to the outer balloon 2 via the fluid supply lumen 111 to inflate the outer balloon 2. Moreover, fluid evacuation can be achieved through the outer-balloon fluid supply lumen 111 to deflate the outer balloon 2. The inner-balloon fluid supply lumen 112 may communicate with the inner balloon 3 to allow a fluid to be supplied to the inner balloon 3 via the fluid supply lumen 112 to inflate the inner balloon 3. Moreover, fluid evacuation can be achieved through the inner-balloon fluid supply lumen 112 to deflate the inner balloon 3.

In this embodiment, the catheter body 1 may include an inner tube 11 and an outer tube 12. The inner tube 11 may be inserted in the outer tube 12, with its distal end extending out of the outer tube 12. A proximal end of the outer balloon 2 may be fixedly attached to the outer tube 12. Also, distal end of the outer balloon may be fixedly attached to the inner tube 11. The inner balloon 3 may be fixedly attached at both its proximal and distal ends to the inner tube 11. Further, the outer-balloon fluid supply lumen 111 may be disposed between the inner tube 11 and the outer tube 12, and the inner-balloon fluid supply lumen 112 may be arranged within the inner tube 11. The inner tube 11 may comprise either one or a plurality of lumens. Preferably, it is a multi-lumen tube provided with, for example, a guidewire lumen, a lead lumen, the fluid supply lumen 112 for the inner balloon and other lumens. A guidewire may be inserted through the guidewire lumen, and the lead lumen may be configured to accommodate electrical leads 5. The catheter body 1 may further include a proximal handle 6, to which both the inner tube 11 and the outer tube 12 may be proximally connected. The handle 6 may be provided with an outer-balloon fluid inlet 61 and an inner-balloon fluid inlet 62. The outer-balloon fluid inlet 61 may connect the outer-balloon fluid supply lumen 111, and the inner-balloon fluid inlet 62 may connect the inner-balloon fluid supply lumen 112. Both the outer-balloon fluid inlet 61 and the inner-balloon fluid inlet 62 may be connected to external fluid sources. The handle 6 may include an energy interface 63, and the electrical leads 5 may be connected to the high-voltage pulse generator through the energy interface 63.

Further, radiopaque structures may be provided at the distal end of the inner tube 11 and within the inner balloon 3. Generally, one radiopaque ring may be provided at each of the proximal and distal ends of the inner balloon to allow locate the inner and outer balloons through X-ray radiography.

The electrode balloon catheter may further include the high-voltage pulse generator, which may be disposed at the proximal end of the catheter body 1. Upon receiving high-voltage pulses from the high-voltage pulse generator, the electrode pairs 4 may generate electrical arcs which vaporize the surrounding conductive medium to form vapor bubbles. These vapor bubbles will expand and eventually burst, generating shock waves which propagate through the conductive medium within the outer balloon 2 to the outer balloon 2 and the wall of the blood vessel surrounding the outer balloon 2 and impinge upon a target calcified lesion. Repeated such pulses can crush the calcified lesion while not causing damage to the vessel wall or surrounding soft tissue. In addition to conducting electricity, the conductive medium can be used to inflate and expand the outer balloon 2. This application is not limited to any particular type of conductive medium. The conductive medium may be a physiological saline solution, conductive hydrogel, conductive antioxidant fluid, contrast fluid or the like. The conductive antioxidant fluid is non-invasive to the electrodes and can enhance durability of the electrodes. In such embodiments, when the outer balloon 2 is advanced to a target lesion site, the conductive medium may be filled into the outer balloon 2. This allows delivery of the outer balloon 2 prior to the inflation in a configuration with a smaller outer diameter and hence enhanced crossability. This configuration may be attained by expelling air from the outer balloon 2 and thus causing its collapse over the exterior of the catheter.

Below, operation of the electrode balloon catheter according to the present embodiment is described.

At first, after being inserted into a blood vessel, the electrode balloon catheter is advanced through a stenotic lesion by virtue of its small outer diameter to a target lesion (i.e., a calcified region). After reaching the target lesion, an amount of a conductive medium is filled into the outer balloon 2 through the outer-balloon fluid supply lumen 111 in communication with the outer balloon 2. The amount and pressure of the filled conductive medium are controlled so that the outer balloon 2 is inflated to a diameter at which the outer balloon 2 completely fits onto the target lesion. Preferably, the conductive medium is radiopaque to X-rays, allowing an operator to observe inflation of the outer balloon 2 and its adherence to the target lesion through X-ray radiography. After the outer balloon 2 is inflated to a desired degree, a volume of a fluid is filled into the inner balloon 3 through the inner-balloon fluid supply lumen 112 in communication with the inner balloon 3. Preferably, the fluid is a contrast agent. The volume of the filled fluid is controlled so that the inner balloon 3 is inflated to a diameter at which the electrode pairs 4 thereon are located at desired positions. After the inner balloon 3 is inflated to a desired degree, the high-voltage pulse generator generates high-voltage pulses, which are then transmitted to the electrode pairs 4 through the electrical leads 5. As a result, high-energy electrons build up on the electrodes and eventually break down the conductive medium between the electrodes in the electrode pairs 4, generating shock waves by the electrohydraulic effect. The resultant shock waves impinge upon and crush the target calcified lesion. At the same time, a pressure is provided through the outer-balloon fluid supply lumen 111 and the outer balloon 2 to dilate a lumen at the lesion. In this way, the electrode balloon catheter can pre-dilate the stenotic lesion and ensure a sufficiently large vascular lumen, allowing subsequent access of a medical device with a relatively large outer diameter to the target lesion, such as a stent delivery device, drug-coated balloon, or the like.

According to the embodiments disclosed herein, the inventive electrode balloon catheter allows adjustability of a distance from the electrodes to target diseased tissue through manipulating the inner balloon. This can reduce energy loss of shock waves during propagation, enhance the performance in crushing a calcified lesion, reduce the surgical duration and cycles of shockwave release, extend the service life of the electrodes and lower the risk of complications. The adjustability of the distance from the electrodes to the target diseased tissue is accomplished by changing a degree of inflation of the inner balloon. This results in higher lesion treatment efficiency and enables the targeted treatment of an asymmetric, eccentric calcified lesion, making the electrode balloon catheter suitable for use in more therapeutic treatment applications and more powerful in therapeutic treatment. As would be appreciated, the electrode balloon catheter of the present invention is particularly suited to use in interventional treatment of coronary artery disease. Of course, it can also be used in interventional treatment of other blood vessel diseases.

As would be appreciated, the above description is merely that of some embodiments of the present invention and is not intended to substantively limit the invention in any way. It is noted that many modifications and additions may be made by those of ordinary skill in the art without departing from the teachings disclosed hereinabove, and such modifications and additions are intended to be included within the scope of the present invention as long as they fall within the wording of the claims.

## Claims

1. An electrode balloon catheter, comprising:
a catheter body (1);
an outer balloon (2), wherein the outer balloon (2) is disposed at an end of the catheter body (1) and is configured to store a conductive medium;
an inner balloon (3), wherein the inner balloon (3) is disposed at the end of the catheter body (1) and is located inside the outer balloon (2); and
at least one electrode pair (4) configured to receive high-voltage pulses so as to generate shock waves, wherein at least one electrode pair (4) is disposed on a surface of the inner balloon (3), wherein the electrode pair (4) comprises a positive electrode and a negative electrode, wherein the positive electrode and the negative electrode are insulated from each other, and wherein an insulating distance between the positive electrode and the negative electrode is fixed or variable, and wherein the insulating distance between the positive and negative electrodes is configured within a predetermined range,
wherein the outer balloon (2), when inflated, is configured to fit onto a target object, wherein the inner balloon (3) is configured to bring the electrode pair (4) closer to or farther away from the target object through an inflation of the inner balloon (3), and wherein the electrode pairs (4) can be adjusted in position with respect to the target lesion by modifying a degree of inflation, and hence a diameter of the inner balloon (3).

2. The electrode balloon catheter according to claim 1, wherein at least one electrode pair (4) is disposed on an outer surface of the inner balloon (3).

3. The electrode balloon catheter according to claim 1, wherein the predetermined range is from 0.01 mm to 10 mm.

4. The electrode balloon catheter according to claim 1, wherein the positive electrode and the negative electrode are rigidly connected, flexibly connected or not connected.

5. The electrode balloon catheter according to claim 1, wherein the electrode pair (4) comprises two electrodes with opposite polarities, wherein each electrode is formed on the surface of the inner balloon (3) by electroplating or is provided in a form of a flexible circuit.

6. The electrode balloon catheter according to claim 1, comprising a plurality of electrode pairs (4), wherein the plurality of electrode pairs (4) are arranged axially along and circumferentially around an inflated inner balloon (3).

7. The electrode balloon catheter according to claim 6, wherein the positive electrode and the negative electrode of each electrode pair (4) are arranged axially along the inflated inner balloon (3), wherein the positive and negative electrodes in adjacent electrode pairs (4) are connected by electrical leads (5).

8. The electrode balloon catheter according to claim 1, wherein the inner balloon (3) is made of a compliant material or a non-compliant material.

9. The electrode balloon catheter according to claim 1, wherein the catheter body (1) comprises an inner tube (11) and an outer tube (12), wherein the inner tube (11) is received in the outer tube (12) and protrudes out of a distal end of the outer tube (12), wherein: each of a proximal end and a distal end of the inner balloon (3) is fixedly attached to the inner tube (11); a proximal end of the outer balloon (2) is fixedly attached to the outer tube (12); a distal end of the outer balloon (2) is fixedly attached to the inner tube (11); an outer-balloon fluid supply lumen (111) in communication with the outer balloon (2) is formed between the inner (11) and outer tubes (12); and an inner-balloon fluid supply lumen (112) in communication with the inner balloon (3) is provided within the inner tube (11).

10. The electrode balloon catheter according to claim 9, wherein the catheter body (1) further comprises a handle (6) that is located at a proximal end, wherein a proximal end of the inner tube (11) and a proximal end of the outer tube (12) are connected to the handle (6), wherein the handle (6) is provided with an outer-balloon fluid inlet (61) and an inner-balloon fluid inlet (62), wherein the outer-balloon fluid inlet (61) is connected to the outer-balloon fluid supply lumen (111), and wherein the inner-balloon fluid inlet (62) is connected to the inner-balloon fluid supply lumen (112).

11. The electrode balloon catheter according to claim 1, wherein the inflated outer balloon (2) has a diameter of 0.75 mm to 30.0 mm and an axial length of 3 mm to 300 mm.

12. The electrode balloon catheter according to claim 1, wherein the inflated inner balloon (3) has a diameter of 0.5 mm to 29.0 mm and an axial length of 3 mm to 300 mm.

## Patentansprüche

1. Elektrodenballonkatheter, der Folgendes umfasst:
einen Katheterkörper (1);
einen äußeren Ballon (2), wobei der äußere Ballon (2) an einem Ende des Katheterkörpers (1) angeordnet ist und zum Speichern eines leitfähigen Mediums konfiguriert ist;
einen inneren Ballon (3), wobei der innere Ballon (3) an dem Ende des Katheterkörpers (1) angeordnet ist und sich innerhalb des äußeren Ballons (2) befindet; und
mindestens ein Elektrodenpaar (4), das zum Empfangen von Hochspannungsimpulsen konfiguriert ist, um Stoßwellen zu erzeugen, wobei mindestens ein Elektrodenpaar (4) auf einer Oberfläche des inneren Ballons (3) angeordnet ist, wobei das Elektrodenpaar (4) eine positive Elektrode und eine negative Elektrode umfasst, wobei die positive Elektrode und die negative Elektrode voneinander isoliert sind und wobei ein Isolationsabstand zwischen der positiven Elektrode und der negativen Elektrode fest oder variabel ist, und wobei der Isolationsabstand zwischen der positiven und der negativen Elektrode innerhalb eines vorbestimmten Bereichs konfiguriert ist,
wobei der äußere Ballon (2) im aufgeblasenen Zustand dazu konfiguriert ist, auf ein Zielobjekt zu passen, wobei der innere Ballon (3) dazu konfiguriert ist, das Elektrodenpaar (4) durch ein Aufblasen des inneren Ballons (3) näher an das Zielobjekt heranzubringen oder davon zu entfernen, und wobei die Position der Elektrodenpaare (4) in Bezug auf die Zielläsion durch Modifizieren eines Aufblasgrades und daher eines Durchmessers des inneren Ballons (3) eingestellt wird.

2. Elektrodenballonkatheter nach Anspruch 1, wobei mindestens ein Elektrodenpaar (4) auf einer Außenoberfläche des inneren Ballons (3) angeordnet ist.

3. Elektrodenballonkatheter nach Anspruch 1, wobei der vorbestimmte Bereich zwischen 0,01 mm und 10 mm liegt.

4. Elektrodenballonkatheter nach Anspruch 1, wobei die positive Elektrode und die negative Elektrode starr verbunden, flexibel verbunden oder nicht verbunden sind.

5. Elektrodenballonkatheter nach Anspruch 1, wobei das Elektrodenpaar (4) zwei Elektroden mit entgegengesetzter Polarität umfasst, wobei jede Elektrode durch Galvanisieren auf der Oberfläche des inneren Ballons (3) durch galvanisches Verzinnen gebildet ist oder in einer Form einer flexiblen Schaltung bereitgestellt wird.

6. Elektrodenballonkatheter nach Anspruch 1, der eine Vielzahl von Elektrodenpaaren (4) umfasst, wobei die Vielzahl von Elektrodenpaaren (4) axial entlang und kreisförmig um einen aufgeblasenen inneren Ballon (3) eingerichtet ist.

7. Elektrodenballonkatheter nach Anspruch 6, wobei die positive Elektrode und die negative Elektrode jedes Elektrodenpaars (4) axial entlang des aufgeblasenen inneren Ballons (3) eingerichtet sind, wobei die positive und die negativen Elektrode in benachbarten Elektrodenpaaren (4) durch elektrische Leitungen (5) verbunden sind.

8. Elektrodenballonkatheter nach Anspruch 1, wobei der innere Ballon (3) aus einem nachgiebigen oder einem nicht nachgiebigen Material hergestellt ist.

9. Elektrodenballonkatheter nach Anspruch 1, wobei der Katheterkörper (1) ein Innenrohr (11) und ein Außenrohr (12) umfasst, wobei das Innenrohr (11) in dem Außenrohr (12) aufgenommen ist und aus einem distalen Ende des Außenrohrs (12) herausragt, wobei: jedes eines proximalen und eines distalen Ende des inneren Ballons (3) fest an dem Innenrohr (11) angebracht ist; ein proximales Ende des äußeren Ballons (2) fest an dem Außenrohr (12) angebracht ist; ein distales Ende des äußeren Ballons (2) fest an dem Innenrohr (11) angebracht ist; ein mit dem äußeren Ballon (2) in Verbindung stehendes Fluidzufuhrlumen (111) des äußeren Ballons zwischen dem Innenrohr (11) und dem Außenrohr (12) gebildet ist; und ein mit dem inneren Ballon (3) in Verbindung stehendes Fluidzufuhrlumen (112) des inneren Ballons innerhalb des Innenrohrs (11) bereitgestellt ist.

10. Elektrodenballonkatheter nach Anspruch 9, wobei der Katheterkörper (1) weiter einen Griff (6) umfasst, der sich an einem proximalen Ende befindet, wobei ein proximales Ende des Innenrohrs (11) und ein proximales Ende des Außenrohrs (12) mit dem Griff (6) verbunden sind, wobei der Griff (6) mit einem Fluideinlass (61) des äußeren Ballons und einem Fluideinlass (62) des inneren Ballons versehen ist, wobei der Fluideinlass (61) des äußeren Ballons mit dem Fluidversorgungslumen (111) des äußeren Ballons verbunden ist, und wobei der Fluideinlass (62) des inneren Ballons mit dem Fluidversorgungslumen (112) des inneren Ballons verbunden ist.

11. Elektrodenballonkatheter nach Anspruch 1, wobei der aufgeblasene äußere Ballon (2) einen Durchmesser von 0,75 mm bis 30,0 mm und eine axiale Länge von 3 mm bis 300 mm aufweist.

12. Elektrodenballonkatheter nach Anspruch 1, wobei der aufgeblasene innere Ballon (3) einen Durchmesser von 0,5 mm bis 29,0 mm und eine axiale Länge von 3 mm bis 300 mm aufweist.

## Revendications

1. Cathéter à ballonnet à électrodes , comprenant :
un corps de cathéter (1) ;
un ballonnet externe (2), dans lequel le ballonnet externe (2) est disposé au niveau d'une extrémité du corps du cathéter (1) et est configuré pour stocker un milieu conducteur ;
un ballonnet interne (3), dans lequel le ballonnet interne (3) est disposé au niveau de l'extrémité du corps du cathéter (1) et est situé à l'intérieur du ballonnet externe (2) ; et
au moins une paire d'électrodes (4) configurée pour recevoir des impulsions haute tension de manière à générer des ondes de choc, dans lequel au moins une paire d'électrodes (4) est disposée sur une surface du ballonnet interne (3), dans lequel la paire d'électrodes (4) comprend une électrode positive et une électrode négative, dans lequel l'électrode positive et l'électrode négative sont isolées l'une de l'autre, et dans lequel une distance d'isolement entre l'électrode positive et l'électrode négative est fixe ou variable, et dans lequel la distance d'isolement entre les électrodes positive et négative est configurée dans une plage prédéterminée,
dans lequel le ballonnet externe (2), une fois gonflé, est configuré pour s'adapter à un objet cible, dans lequel le ballonnet interne (3) est configuré pour rapprocher ou éloigner la paire d'électrodes (4) de l'objet cible à travers un gonflage du ballonnet interne (3), et dans lequel les paires d'électrodes (4) peuvent être ajustées en position par rapport à la lésion cible en modifiant un degré de gonflage, et donc un diamètre du ballonnet interne (3).

2. Cathéter à ballonnet à électrodes selon la revendication 1, dans lequel au moins une paire d'électrodes (4) est disposée sur une surface externe du ballonnet interne (3).

3. Cathéter à ballonnet à électrodes selon la revendication 1, dans lequel la plage prédéterminée est comprise entre 0,01 mm et 10 mm.

4. Cathéter à ballonnet à électrodes selon la revendication 1, dans lequel l'électrode positive et l'électrode négative sont connectées de manière rigide, connectées de manière flexible ou non connectées.

5. Cathéter à ballonnet à électrodes selon la revendication 1, dans lequel la paire d'électrodes (4) comprend deux électrodes avec des polarités opposées, dans lequel chaque électrode est formée sur la surface du ballonnet interne (3) par galvanoplastie ou étant fournie sous la forme d'un circuit flexible.

6. Cathéter à ballonnet à électrodes selon la revendication 1, comprenant une pluralité de paires d'électrodes (4), dans lequel la pluralité de paires d'électrodes (4) sont disposées axialement le long et circonférentiellement autour d'un ballonnet interne gonflé (3).

7. Cathéter à ballonnet à électrodes selon la revendication 6, dans lequel l'électrode positive et l'électrode négative de chaque paire d'électrodes (4) sont disposées axialement le long du ballonnet interne gonflé (3), dans lequel les électrodes positives et négatives dans des paires d'électrodes adjacentes (4) sont connectées par des fils électriques (5).

8. Cathéter à ballonnet à électrodes selon la revendication 1, dans lequel le ballonnet interne (3) est constitué d'un matériau souple ou d'un matériau non souple.

9. Cathéter à ballonnet à électrodes selon la revendication 1, dans lequel le corps de cathéter (1) comprend un tube interne (11) et un tube externe (12), dans lequel le tube interne (11) est reçu dans le tube externe (12) et fait saillie en dehors d'une extrémité distale du tube externe (12), dans lequel : chacune d'une extrémité proximale et d'une extrémité distale du ballonnet interne (3) est solidement fixée au tube interne (11) ; une extrémité proximale du ballonnet externe (2) est solidement fixée au tube externe (12) ; une extrémité distale du ballonnet externe (2) est solidement fixée au tube interne (11) ; une lumière d'alimentation en fluide de ballonnet externe (111) en communication avec le ballonnet externe (2) est formée entre les tubes interne (11) et externe (12) ; et une lumière d'alimentation en fluide de ballonnet interne (112) en communication avec le ballonnet interne (3) est prévue dans le tube interne (11).

10. Cathéter à ballonnet à électrodes selon la revendication 9, dans lequel le corps de cathéter (1) comprend en outre une poignée (6) qui est située au niveau d'une extrémité proximale, dans lequel une extrémité proximale du tube interne (11) et une extrémité proximale du tube externe (12) sont connectées à la poignée (6), dans lequel la poignée (6) est pourvue d'une entrée de fluide de ballonnet externe (61) et d'une entrée de fluide de ballonnet interne (62), dans lequel l'entrée de fluide de ballonnet externe (61) est connectée à la lumière d'alimentation en fluide de ballonnet externe (111), et dans lequel l'entrée de fluide de ballonnet interne (62) est connectée à la lumière d'alimentation en fluide de ballonnet interne (112).

11. Cathéter à ballonnet à électrodes selon la revendication 1, dans lequel le ballonnet externe gonflé (2) a un diamètre de 0,75 mm à 30,0 mm et une longueur axiale de 3 mm à 300 mm.

12. Cathéter à ballonnet à électrodes selon la revendication 1, dans lequel le ballonnet interne gonflé (3) a un diamètre de 0,5 mm à 29,0 mm et une longueur axiale de 3 mm à 300 mm.
